# FASCICULE DE BREVET EUROPEEN

(11) **EP 2 485 683 B1**
(45) Date de publication et mention de la délivrance du brevet: **31.07.2013**
(21) Numéro de dépôt: 10778693.1
(22) Date de dépôt: 04.10.2010
(51) Int. Cl.: B28B 1/00, C04B 38/00, B29C 67/00, A61F 2/14

(54) **BILLE À ÉNUCLÉATION ET PROCÉDÉ DE FABRICATION D'UNE TELLE BILLE**
ENUKLEATIONSKUGEL UND VERFAHREN ZUR HERSTELLUNG SOLCH EINER KUGEL
ENUCLEATION BALL AND METHOD FOR MANUFACTURING SUCH A BALL

(30) Priorité: 08.10.2009 FR 0957032
(43) Date de publication de la demande: 15.08.2012
(73) Titulaire: Kasios, 31140 Launaguet (FR)
(72) Inventeur: GUENA, Nicolas, F-75016 Paris (FR)
(74) Mandataire: Cabinet HERRBURGER
(86) Numéro de dépôt international: PCT/FR2010/052090
(87) Numéro de publication internationale: WO 2011/042650

(56) Documents cités:
- WO-A2-2006/083875
- US-A- 5 584 880
- US-A- 5 733 332
- US-A1- 2005 125 060

## Description

### Domaine de l'invention

La présente invention concerne une bille à énucléation réalisée en matière céramique.

L'invention concerne également un procédé de fabrication d'une telle bille à énucléation.

### Etat de la technique

En chirurgie, il est connu depuis longtemps de remplacer le globe oculaire extrait de la cavité orbitaire par une bille afin remplir le volume laissé libre. Cette intervention est réalisée à des fins cosmétiques. Le devant de cette bille reçoit, ensuite, un « oeil de verre » ayant la forme d'une demi-coque et sur laquelle est reproduit le dessin de l'oeil.

Cette bille peut être fabriquée dans différents matériaux comme l'ivoire, le verre ou le plastique (PMMA). Aujourd'hui, seules les billes en plastique sont encore utilisées.

Ces billes pleines fabriquées dans un matériau dur présentent l'inconvénient de ne pas permettre la suture directe des muscles oculaires à la bille. Pour raccorder les muscles à de telles billes, le chirurgien doit au préalable envelopper les billes soit avec de la sclère d'origine humaine soit avec un treillis synthétique. Mais cette manoeuvre est longue et rallonge le temps de l'intervention.

Des billes en PMMA ont aussi été fabriquées avec quatre tunnels positionnés au tiers supérieur de la sphère afin de permettre le passage des aiguilles de suture et de fixer les muscles à la bille. L'inconvénient de ce système tient à ce que les muscles doivent toujours être fixés au même endroit ce qui n'est pas toujours réalisable car certains muscles peuvent être atrophiés.

C'est pourquoi, plus récemment et pendant toute une période, les billes à énucléation ont été fabriquées en silicone, cette matière est suffisamment souple pour permettre au chirurgien de suturer les muscles directement dessus en piquant les aiguilles dans le silicone.

Ces billes ont connu un certain succès mais elles ont ensuite été supplantées par des billes poreuses fabriquées en céramique d'hydroxyapatite (HA) et ou en céramique d'alumine (Al2O3). Le grand avantage de ces billes poreuses tient à ce qu'elles sont réhabitées par les tissus du patient. On obtient ainsi une bille envahie par un tissu fibro-vasculaire. Cette bille, une fois recolonisée par les tissus du patient, est rendue plus mobile dans l'orbite ce qui permet une meilleure restitution des mouvements oculaires.

Les billes en céramique d'hydroxyapatite ou en céramique d'alumine ont comme inconvénient d'être fabriquées dans un matériau dur à travers lequel on ne peut pas passer les aiguilles des sutures. On est donc obligé de les envelopper, comme on le faisait avec les billes en verre ou en PMMA, afin de pouvoir les relier aux muscles oculaires.

Une autre bille en polyéthylène poreux est suffisamment souple pour permettre la suture des muscles directement et à n'importe quel endroit de la bille. L'inconvénient de cette bille est sa faible porosité qui n'autorise qu'une réhabitation très partielle et très superficielle par les tissus du receveur. US 2005/125060 décrit une bille à énucléation se composant d'une sphère intérieure poreuse en céramique, portant une sphère extérieure.

### But de l'invention

La présente invention a pour but de développer une bille poreuse en céramique d'hydroxyapatite ou d'alumine sur laquelle on pourra suturer les muscles directement et à n'importe quel endroit et pouvant être implantée indifféremment au cours d'une énucléation ou au cours d'une éviscération.

### Exposé et avantages de l'intention

A cet effet, l'invention concerne une bille à énucléation du type défini ci-dessus caractérisée en ce qu'elle se compose d'une sphère intérieure poreuse portant une sphère extérieure ajourée, par l'intermédiaire d'entretoises reliant la sphère intérieure à la sphère extérieure.

La bille à énucléation selon l'invention offre le double avantage d'être en céramique poreuse permettant la réhabitation complète avec les tissus du receveur et la suture des muscles aux endroits les plus appropriés pour chaque cas d'intervention. Les sutures se font directement sur la sphère extérieure, l'intervalle entre la sphère intérieure et la sphère extérieure permettant le passage des aiguilles de suture. La sphère extérieure et la sphère intérieure offrent des surfaces de réhabitation efficaces et la combinaison de la sphère intérieure et de la sphère extérieure assure à la bille, la tenue mécanique tout en constituant une bille légère.

Suivant une autre caractéristique avantageuse, la sphère extérieure est une surface ajourée formée par des branches méridiennes se coupant selon l'axe de la bille et croisant des branches en cercles contenus dans des plans perpendiculaires à l'axe des branches méridiennes.

Cette structure de la sphère extérieure permet de créer un réseau dense pratiquement continu sur toute la surface de la bille de possibilités de points de suture.

Suivant une autre caractéristique, l'un des pôles de la sphère extérieure forme une calotte non ajourée ou suivant une autre caractéristique, l'un des pôles de la sphère extérieure est ouvert en forme de calotte ajourée, bordée par un cercle.

Mais on peut également combiner ces deux solutions, c'est-à-dire avoir une bille dont les deux pôles ont une calotte non ajourée ou les deux pôles ont une calotte ajourée.

Suivant une autre caractéristique, les entretoises sont dirigées radialement à partir des intersections de plusieurs branches bordant les jours de la sphère extérieure.

L'invention concerne également un procédé de fabrication d'une bille à énucléation en céramique poreuse caractérisé en ce qu'
- on définit la géométrie de la sphère intérieure, de la sphère extérieure et des entretoises par une définition numérique des sphères, de l'épaisseur de la matière, de la largeur des branches, de la forme et de la dimension des trous de la sphère intérieure,
- on découpe sa forme géométrique en tranches par des plans parallèles, puis,
- avec un appareil de stéréolithographie, on construit la bille couche par couche correspondant à des plans de coupe parallèle selon la géométrie de la bille qui a été définie, en utilisant une matière plastique non polymérisée mais polymérisable par action de la chaleur et chargée d'une poudre de céramique,
- on polymérise successivement une couche après l'autre une fois la bille complètement réalisée avec un faisceau laser pour fixer provisoirement la matière plastique chargée de poudre de matière céramique,
- on élimine la matière plastique non polymérisée et
- on cuit la bille ainsi obtenue pour éliminer la matière plastique polymérisée et fritter la matière céramique de la bille.

L'invention concerne également un procédé de fabrication directe d'une bille à énucléation, ce procédé étant caractérisé en ce qu'
- on définit la géométrie de la sphère intérieure, de la sphère extérieure et des entretoises par une définition numérique des sphères, de l'épaisseur de la matière, de la largeur des branches, de la forme et de la dimension des trous de la sphère intérieure,
- on découpe sa forme géométrique en tranches par des plans parallèles, puis,
- avec un appareil de stéréolithographie, on construit la bille couche par couche correspondant à des plans de coupe parallèle selon la géométrie de la bille qui a été définie, en utilisant une matière plastique non polymérisée mais polymérisable par action de la chaleur et chargée d'une poudre de céramique,
- on polymérise avec un faisceau laser de puissance suffisante pour fixer provisoirement la matière plastique chargée de poudre de matière céramique puis détruire la matière plastique et cuire en même temps la poudre céramique pour la fritter.

La poudre céramique servant à fabriquer la bille à énucléation est, de préférence, une poudre d'alumine ou d'hydroxyapatite.

### Dessins

La présente invention sera décrite ci-après de manière plus détaillée à l'aide d'exemples de réalisation représentés dans les dessins annexés dans lesquels :
- la figure 1 est une vue isométrique de dessus d'un exemple de bille à énucléation selon l'invention,
- la figure 2 est une vue isométrique de dessous de la bille à énucléation de la figure 1,
- la figure 3A est une vue de dessus de la bille de la figure 1, coupée par un plan perpendiculaire à l'axe et correspondant à une phase de fabrication de la bulle,
- la figure 3B est une vue en coupe schématique par un plan méridien de la bille de la figure 1 en fin de fabrication.

### Description de modes de réalisation de l'invention

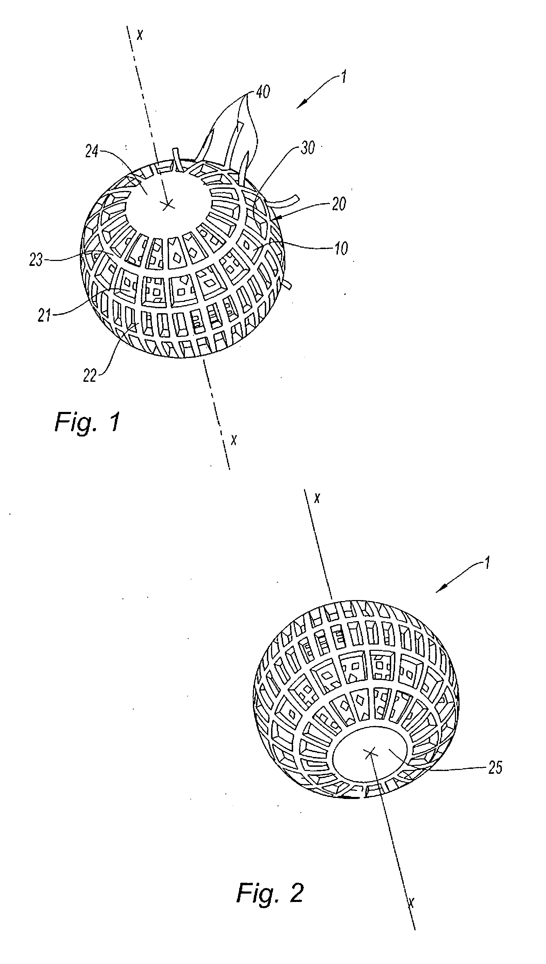

Selon les figures 1 et 2, l'invention concerne une bille à énucléation 1 en matière céramique telle que de l'alumine Al₂O₃ ou de l'hydroxyapatite HA, composée d'une sphère intérieure 10 poreuse et d'une sphère extérieure 20 ajourée, les deux sphères réalisée dans la même matière et en une seule pièce, étant reliées par des entretoises 30 transmettant, les efforts exercés sur la sphère extérieure 20.

La sphère extérieure 20 ayant le diamètre usuel d'une bille à énucléation, est ajourée, de préférence, régulièrement sur toute sa surface selon un motif à symétrie circulaire autour de son axe XX.

Selon ce mode de réalisation, la surface ajourée est formée d'un réseau de branches 21, 22 appartenant à des cercles méridiens d'axe XX et des branches 12 formées par des cercles dans des plans perpendiculaires à l'axe XX. L'un des pôles 24 de la sphère est une calotte non ajourée et l'autre pôle 25, ajouré, est formé d'un orifice bordé par un cercle polaire 26 où se rejoignent les branches 21, 22 des cercles méridiens dont les autres extrémités partent de la calotte non ajourée 24.

Pour laisser des jours de dimensions suffisantes vers les pôles de la sphère extérieure 20, certaines des branches méridiennes 22 n'occupent qu'une partie de la sphère extérieure 20 au niveau de sa zone équatoriale.

Les entretoises 30 se situent de préférence à l'intersection des branches méridiennes 21, 22 et des cercles 23 et elles sont dirigées radialement.

Selon une forme de réalisation, les deux pôles 24, 25 sont ajourés.

Selon une autre forme de réalisation non représentée, les branches délimitant les jours de la sphère extérieure 20 sont constituées par des courbes décrivant la surface de la sphère extérieure et notamment des réseaux de loxodromies, par exemple deux réseaux d'angles opposés par rapport à un plan méridien et qui remplacent les cercles méridiens et les cercles dans des plans perpendiculaires à l'axe XX de la bille 1.

Les jours délimités entre les branches 21, 22, 13 de la sphère extérieure 20 ont des dimensions permettant le passage d'aiguilles de suture 40 pratiquement en n'importe quel endroit de la sphère extérieure, sauf au niveau de la calotte 24. Des exemples d'aiguilles de suture 40 dans différentes positions sont représentés à la figure 1.

La sphère extérieure 20 est séparée de la sphère intérieure 10 d'un intervalle d'épaisseur fixé par les entretoises 30 et défini de manière à permettre le passage des aiguilles de suture 10 traversant la sphère extérieure 20 et ressortant de celle-ci après passage dans l'intervalle.

La sphère intérieure 10 combine une macroporosité et une microporosité : sa macroporosité est réalisée par des trous 11 aptes à permettre la réhabitation, favorisée en outre par la microporosité de sa matière céramique. Les trous 11 sont de dimensions suffisamment petites pour ne pas être traversés par l'aiguille de suture 40. Ainsi, la sphère intérieure 10 a, outre la fonction de réhabitation, d'autres fonctions et notamment celle de guider et de renvoyer l'aiguille de suture 40 vers la sphère extérieure 20 pour qu'elle la retraverse ; elle a aussi une fonction de renforcement de la sphère extérieure 20.

La réhabitation de la bille 1 se fait en partie sur la sphère extérieure 20 qui bénéficie de la microporosité de sa matière céramique. Mais comme les jours de la sphère extérieure 20 peuvent être trop grands pour permettre une réhabitation rapide, la réhabitation se développera sur la sphère intérieure 10 grâce à sa macroporosité complétée par la microporosité de sa matière.

Selon les praticiens et les techniques qu'ils appliquent, la bille 1 sera installée avec la calotte non ajourée 24 tournée vers l'avant ou la partie de calotte ajourée 25, tournée vers l'avant, suivant les emplacements où ils veulent fixer les muscles optiques. Ensuite, le pôle avant de la sphère est couvert par une demi-coquille avec le dessin de l'oeil. Cet élément n'est pas représenté.

La bille 1 selon l'invention, se fabrique de préférence, indirectement ou directement, par stéréolithographie.

Selon le procédé indirect, après avoir défini la géométrie de la sphère intérieure 10, de la sphère extérieure 20 et des entretoises 30 reliant les deux sphères, on découpe sa forme géométrique en tranches par des plans parallèles, par exemple et de préférence, des couches perpendiculaires à l'axe XX de manière à bénéficier de la symétrie de rotation de la forme de bille autour de son axe XX. Ensuite, on utilise un appareil de stéréolithographie pour construire la bille, couche par couche. Dans l'appareil de stéréolithographie, l'axe devient un axe vertical.

Le développement de la bille se fait par couches successives d'une résine polymérisée sous l'effet de la lumière et de la chaleur suivant le tracé des différentes parties de la bille dans les plans de coupe

La résine utilisée contient des poudres céramiques d'alumine ou de hydroxyapatite. L'appareil de photolithographie réalise les couches successives du produit en deux dimensions composant, par leur empilage, la forme tridimensionnelle des deux sphères et des entretoises de la bille.

Pour cela, chaque couche de la bille est réalisée sur la couche précédente à l'aide d'un faisceau laser qui balaie le tracé de la couche pour faire polymériser le liquide emprisonnant des particules de matière céramique.

Lorsque la bille est terminée, on évacue la résine non polymérisée à travers les trous de la sphère intérieure 10 et de la sphère extérieure 20. On peut dissoudre la résine dans un solvant de façon à recueillir l'objet formé en résine polymérisée emprisonnant les particules de céramique.

Au cours de l'étape suivante, on cuit la bille ainsi produite pour éliminer la résine polymérisée et ne conserver que les particules de céramique que l'on fritte par la même opération.

La bille est ainsi terminée.

Selon le procédé direct, on utilise un laser plus puissant que celui servant à polymériser les couches successives de matière plastique emprisonnant la poudre céramique pour fritter directement la poudre céramique en couches successives constituant alors directement la bille avec ses deux sphères 10, 20 et les entretoises 30.

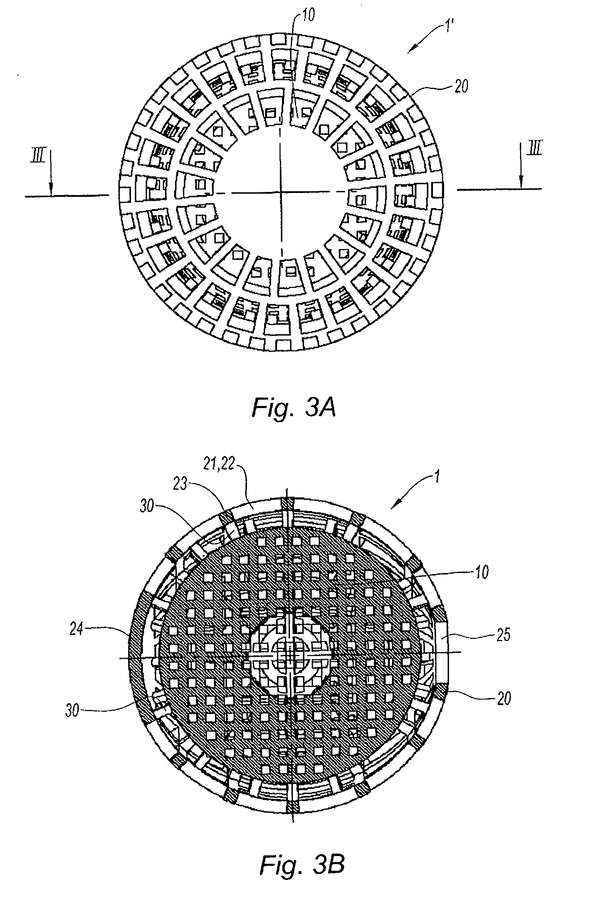

La figure 3A montre, en plan horizontal, une bille 1 en cours de formation et la figure 3B, la bille terminée en coupe verticale.

La figure 3A montre la bille dont la partie inférieure (selon la vue de la figure 3A) est terminée et la fabrication se poursuit par le dessus de la bille, par exemple vers la calotte 24. Les cercles 23 sont fabriqués et les branches 21 sont en cours de fabrication. Il en est de même de la sphère intérieure 10 qui est encore ouverte.

Au fur et à mesure de l'empilage des couches, les branches 21 s'allongent pour rejoindre le pôle de la sphère extérieure 20 qui est par exemple sous forme de calotte 24 (figure 1) ou sous forme de pôle ouvert 25 bordé par un cercle 26 comme à la figure 2. La sphère intérieure 10 sera fermée progressivement.

Cette figure ne montre pas les trous 11 dans la sphère intérieure 10. Ces trous sont réalisés automatiquement, selon le tracé qu'ils ont dans les différents plans de coupe superposés.

La figure 3B donne schématiquement une vue en coupe par le plan III-1II de la figure 3A correspondant à un plan méridien mais de la bille terminée.

La réalisation présentée aux figures 1-3B correspondant tant à la fabrication indirecte d'une bille qu'à la fabrication directe, les deux procédés se distinguant comme indiqué ci-dessus en ce qu'à la fin du procédé indirect, il faut cuire la bille et la fritter alors que dans le cas du procédé direct, la bille est immédiatement frittée lors de la réalisation des couches successives qui la composent.

### NOMENCLATURE

- 1: bille
- 1': bille en cours de formation
- 10: sphère intérieure
- 11: trous de la sphère intérieure
- 20: sphère extérieure
- 21, 22, 23: branches de la sphère extérieure
- 24: pôle / calotte non ajourée
- 25: pôle ouvert / calotte ajourée
- 26: cercle
- 30: entretoise
- 40: aiguille de suture
- XX: axe de la bille / sphère

## Revendications

1. Bille à énucléation réalisée en matière céramique,
composée d'une sphère intérieure (10) poreuse portant une sphère extérieure (20) ajourée, par l'intermédiaire d'entretoises (30) reliant la sphère intérieure à la sphère extérieure.

2. Bille selon la revendication 1,
**caractérisée en ce que**
la sphère extérieure (20) est une surface ajourée formée par des branches méridiennes (21, 22) se coupant selon l'axe (XX) de la bille et croisant des branches (23) en cercles contenus dans des plans perpendiculaires à l'axe (XX) des branches méridiennes (21, 22).

3. Bille selon la revendication 1,
**caractérisée en ce que**
l'un des pôles (24) de la sphère extérieure (20) forme une calotte non ajourée.

4. Bille selon la revendication 1,
**caractérisée en ce que**
l'un des pôles (26) de la sphère extérieure (20) est ouvert en forme de calotte ajourée, bordée par un cercle (26).

5. Bille selon la revendication 2,
**caractérisée en ce que**
les entretoises sont dirigées radialement à partir des intersections de plusieurs branches bordant les jours de la sphère extérieure (20).

6. Procédé de fabrication d'une bille à énucléation selon l'une des revendications 1 à 5,
**caractérisé en ce qu'**
- on définit la géométrie de la sphère intérieure (10), de la sphère extérieure (20) et des entretoises (30) par une définition numérique des sphères (10, 20), de l'épaisseur de la matière, de la largeur des branches (21, 22, 23), de la forme et de la dimension des trous (11) de la sphère intérieure (10),
- on découpe sa forme géométrique en tranches par des plans parallèles, puis,
- avec un appareil de stéréolithographie, on construit la bille (1) couche par couche correspondant à des plans de coupe parallèle selon la géométrie de la bille qui a été définie, en utilisant une matière plastique non polymérisée mais polymérisable par action de la chaleur et chargée d'une poudre de céramique,
- on polymérise successivement une couche après l'autre une fois la bille complètement réalisée avec un faisceau laser pour fixer provisoirement la matière plastique chargée de poudre de matière céramique,
- on élimine la matière plastique non polymérisée et
- on cuit la bille ainsi obtenue pour éliminer la matière plastique polymérisée et fritter la matière céramique de la bille.

7. Procédé de fabrication d'une bille à énucléation selon les revendications 1 à 5,
**caractérisé en ce qu'**
- on définit la géométrie de la sphère intérieure (10), de la sphère extérieure (20) et des entretoises (30) par une définition numérique des sphères (10, 20), de l'épaisseur de la matière, de la largeur des branches (21, 22, 23), de la forme et de la dimension des trous (11) de la sphère intérieure (10),
- on découpe sa forme géométrique en tranches par des plans parallèles, puis,
- avec un appareil de stéréolithographie, on construit la bille (1) couche par couche correspondant à des plans de coupe parallèle selon la géométrie de la bille qui a été définie, en utilisant une matière plastique non polymérisée mais polymérisable par action de la chaleur et chargée d'une poudre de céramique,
- on polymérise avec un faisceau laser de puissance suffisante pour fixer provisoirement la matière plastique chargée de poudre de matière céramique puis détruire la matière plastique et cuire en même temps la poudre céramique pour la fritter.

8. Procédé selon l'une des revendications 6 ou 7,
**caractérisé en ce qu'**
on utilise une poudre céramique d'alumine ou d'hydroxyapatite pour la réalisation de la bille.

## Patentansprüche

1. Enukletationskugel, die aus Keramikmaterial hergestellt ist, bestehend aus einer porösen Innenkugel (10), die eine durchbrochene Außenkugel (20) über Stege (30) trägt, welche die Innenkugel mit der Außenkugel verbinden.

2. Enukleationskugel nach Anspruch 1,
**dadurch gekennzeichnet, dass**
die Außenkugel (20) eine durchbrochene Fläche ist, die aus meridional verlaufenden Schenkeln (21, 22) gebildet ist, die sich in der Achse (XX) der Enukleationskugel schneiden und kreisförmige Schenkel (23) kreuzen, die in sich senkrecht zur Achse (XX) der meridional verlaufenden Schenkeln (21, 22) erstreckenden Ebenen enthalten sind.

3. Enukleationskugel nach Anspruch 1,
**dadurch gekennzeichnet, dass**
einer der Pole (24) der Außenkugel (20) eine nicht durchbrochene Kalotte bildet.

4. Enukleationskugel nach Anspruch 1,
**dadurch gekennzeichnet, dass**
einer der Pole (26) der Außenkugel (20) in Form einer durchbrochenen Kalotte geöffnet ist, die von einem Kreis (26) umrandet wird.

5. Enukleationskugel nach Anspruch 2,
**dadurch gekennzeichnet, dass**
die Stege ausgehend von den Schnittpunkten mehrerer Schenkel, welche die Durchbrechungen der Außenkugel (20) umranden, radial gerichtet sind.

6. Verfahren zum Herstellen einer Enukleationskugel nach einem der Ansprüche 1 bis 5,
**dadurch gekennzeichnet, dass**
- die Geometrie der Innenkugel (10), der Außenkugel (20) und der Stege (30) durch eine digitale Definition der Kugeln (10, 20), der Materialstärke, der Breite der Schenkel (21, 22, 23), der Form und der Abmessung der Löcher (11) der Innenkugel (10) definiert wird,
- deren geometrische Form durch parallel verlaufende Ebenen in Scheiben zerschnitten wird, wonach
- mit einer stereolithographischen Einrichtung die Enukleationskugel (1) Schicht für Schicht konstruiert wird, die parallel verlaufenden Schnittebenen entlang der Geometrie der Kugel entsprechen, die definiert wurde, indem ein Kunststoffmaterial verwendet wird, das nicht polymerisiert ist, jedoch durch Wärmeeinwirkung polymerisierbar ist und mit Keramikpulver versehen ist,
- nacheinander eine Schicht nach der anderen polymerisiert wird, nachdem die Enukleationskugel vollständig hergestellt wurde, und zwar mit einem Laserstrahl, um das keramikpulverhaltige Kunststoffmaterial vorübergehend zu fixieren,
- das nicht polymerisierte Kunststoffmaterial entfernt wird und
- die so erhaltene Enukleationskugel ausgehärtet wird, um das polymerisierte Kunststoffmaterial zu entfernen und das Keramikmaterial der Enukleationskugel zu sintern.

7. Verfahren zum Herstellen einer Enukleationskugel nach den Ansprüchen 1 bis 5,
**dadurch gekennzeichnet, dass**
die Geometrie der Innenkugel (10), der Außenkugel (20) und der Stege (30) durch eine digitale Definition der Kugeln (10, 20), der Materialstärke, der Breite der Schenkel (21, 22, 23), der Form und der Abmessung der Löcher (11) der Innenkugel (10) definiert wird,
- deren geometrische Form durch parallel verlaufende Ebenen in Scheiben zerschnitten wird, wonach
- mit einer stereolithographischen Einrichtung die Enukleationskugel (1) Schicht für Schicht konstruiert wird, die parallel verlaufenden Schnittebenen entlang der Geometrie der Kugel entsprechen, die definiert wurde, indem ein Kunststoffmaterial verwendet wird, das nicht polymerisiert ist, jedoch durch Wärmeeinwirkung polymerisierbar ist und mit Keramikpulver versehen ist,
- mit einem Laserstrahl mit ausreichender Leistung polymerisiert wird, um das keramikpulverhaltige Kunststoffmaterials vorübergehend zu fixieren und dann das Kunststoffmaterial zu zerstören und gleichzeitig das Keramikpulver auszuhärten, um es zu sintern.

8. Verfahren nach einem der Ansprüche 6 oder 7,
**dadurch gekennzeichnet, dass**
ein Aluminiumoxid- oder Hydroxylapatit-Keramikpulver zum Herstellen der Enukleationskugel verwendet wird.

## Claims

1. Enucleation ball produced from ceramic material,
composed of a porous inner sphere (10) which carries an openwork outer sphere (20) by means of cross-members (30) which connect the inner sphere to the outer sphere.

2. Ball according to claim 1,
**characterised in that**
the outer sphere (20) is an openwork surface formed by meridian branches (21, 22) which intersect along the axis (XX) of the ball and which cross branches (23) in continuous circles in planes perpendicular relative to the axis (XX) of the meridian branches (21, 22).

3. Ball according to claim 1,
**characterised in that**
one of the poles (24) of the outer sphere (20) forms a non-openwork cap.

4. Ball according to claim 1,
**characterised in that**
one of the poles (26) of the outer sphere (20) is open in the form of an openwork cap, bordered by a circle (26).

5. Ball according to claim 2,
**characterised in that**
the cross-members are directed radially from the intersections of a plurality of branches which border the apertures of the outer sphere (20).

6. Method for producing an enucleation ball according to any one of claims 1 to 5,
**characterised in that**
- the geometry of the inner sphere (10), the outer sphere (20) and the gaps (30) is defined by means of a digital definition of the spheres (10, 20), the thickness of the material, the width of the branches (21, 22, 23), the shape and the dimension of the holes (11) of the inner sphere (10),
- the geometric shape thereof is cut into slices by means of parallel planes, then,
- using a stereolithography machine, the ball (1) is constructed layer by layer corresponding to parallel cutting planes in accordance with the geometry of the ball which has been defined, using a plastics material which is non-polymerised but which can be polymerised by the action of heat and which is charged with a ceramic powder,
- one layer after the other is successively polymerised, after the ball has been completely produced with a laser beam in order to provisionally fix the plastics material charged with ceramic material powder,
- the non-polymerised plastics material is eliminated and
- the ball thus obtained is baked in order to eliminate the polymerised plastics material and to sinter the ceramic material of the ball.

7. Method for producing an enucleation ball according to claims 1 to 5,
**characterised in that**
- the geometry of the inner sphere (10), the outer sphere (20) and the cross-members (30) is defined by means of a digital definition of the spheres (10, 20), the thickness of the material, the width of the branches (21, 22, 23), the shape and the dimension of the holes (11) of the inner sphere (10),
- the geometric shape thereof is cut into slices by means of parallel planes, then,
- using a stereolithography machine, the ball (1) is constructed layer by layer corresponding to parallel cutting planes in accordance with the geometry of the ball which has been defined, using a plastics material which is non-polymerised but which can be polymerised by the action of heat and which is charged with a ceramic powder,
- the polymerisation is carried out with a laser beam having sufficient power to provisionally fix the plastics material charged with ceramic material powder, then to destroy the plastics material and to bake at the same time the ceramic powder in order to sinter it.

8. Method according to either claim 6 or claim 7,
**characterised in that**
a ceramic powder of alumina or hydroxyapatite is used in order to produce the ball.
